# FASCICULE DE BREVET EUROPEEN

(11) **EP 1 707 181 B1**
(45) Date de publication et mention de la délivrance du brevet: **01.10.2008**
(21) Numéro de dépôt: 06111841.0
(22) Date de dépôt: 28.03.2006
(51) Int. Cl.: A61K 8/34, A61K 8/39, A61K 8/73, A61K 8/81, A61K 8/87, A61Q 5/06, A61Q 5/10

(54) **Composition colorante à teneur diminuée en matières premières et procédé de coloration de fibres kératiniques la mettant en oeuvre**
Haarfärbemittel mit reduziertem Gehalt an Rohstoffe und das Verfahren zum Färben von Keratinfasern
Dye composition with a reduced content of starting materials, and process for dyeing keratin fibres using the same

(30) Priorité: 31.03.2005 FR 0550835
(43) Date de publication de la demande: 04.10.2006
(73) Titulaire: L'ORÉAL, 75008 Paris (FR)
(72) Inventeur: Legrand, Frédéric, Shinjuku-ku 162-0842 Tokyo (JP)
(74) Mandataire: Fevrier, Murielle Françoise E.

(56) Documents cités:
- EP-A- 1 426 032
- EP-A- 1 428 506
- EP-A- 1 473 025
- WO-A-02/45674

## Description

La présente invention a pour objet une composition colorante comprenant une teneur en eau d'au moins 40 % en poids, ainsi qu'un procédé de coloration de fibres kératiniques, notamment humaines, mettant en oeuvre une telle composition. Elle concerne de même un dispositif à plusieurs compartiments comprenant d'une part la composition colorante et d'autre part une composition oxydante.

Il existe essentiellement deux types de coloration des fibres kératiniques, notamment humaines, telles que les cheveux.

La première, appelée coloration d'oxydation ou encore coloration permanente, consiste à mettre en oeuvre des précurseurs de colorants d'oxydation, qui sont des substances peu ou non colorées. Lorsqu'ils sont mis en présence d'un agent oxydant, ces composés produisent, par un processus de condensation oxydative ayant lieu à l'intérieur même de la fibre, des substances colorées qui restent piégées dans la fibre.

La seconde, appelée coloration directe ou encore semi-permanente, est obtenue en mettant en oeuvre des composés colorés et colorants ayant une affinité avec les fibres kératiniques sur lesquelles ils sont appliqués. Ce type de coloration ne nécessite pas de mettre en oeuvre un agent oxydant pour révéler la couleur, bien qu'il ne soit pas exclu que ce type d'agent soit présent pendant le procédé. On parle alors dans ce dernier cas de coloration directe éclaircissante.

Les compositions colorantes de l'état de la technique se présentent dans la majorité des cas, sous la forme de liquides, de gels ou de crèmes qui sont, si nécessaire, mélangés avant l'application sur les fibres, à une composition oxydante.

Les compositions colorantes sont le plus souvent relativement riches en matières premières, parmi lesquelles on trouve habituellement des corps gras, des tensioactifs et/ou des polymères. Ces compositions sont formulées de telle sorte qu'elles présentent des propriétés d'étalement et des textures faciles à travailler afin de permettre une application aisée et rapide sur les fibres, tout en étant suffisamment épaisses pour ne pas couler hors des zones que l'on souhaite colorer. De plus ces compositions doivent rester stables pendant le temps de pause sur les fibres et être facilement éliminables au rinçage une fois la coloration obtenue.

Or il n'est pas rare de constater que des quantités importantes de matières premières pénalisent les performances colorantes de telles compositions. On peut ainsi observer une cinétique moins favorable, une intensité amoindrie de la nuance obtenue, une moins bonne homogénéité de la couleur d'une fibre à l'autre et/ou selon l'endroit de la fibre (racine/pointe), etc.

La présente invention a donc pour objet de proposer des compositions colorantes, qui ne présentent pas les inconvénients ci-dessus mentionnés des compositions colorantes actuelles, tout en conservant les propriétés citées ci-dessus.

Un tel objectif est atteint par la présente invention qui a donc pour objet des compositions colorantes comprenant, dans un milieu approprié pour la teinture des fibres kératiniques :
- au moins un précurseur de colorant d'oxydation et/ou au moins un colorant direct ;
- au moins un tensioactif non ionique, anionique ou leurs mélanges ;
- au moins l'hydroxyéthylcellulose ;
- au moins un polymère associatif cationique ;
- au moins un corps gras ;
- le rapport pondéral de la quantité totale de tensioactifs non ioniques et anioniques à la quantité totale en corps gras étant supérieur ou égal à 1,75 ; et
- la teneur en eau dans la composition colorante représentant au moins 40 % en poids par rapport au poids de ladite composition colorante.

L'invention a de plus pour objet un procédé de coloration de fibres kératiniques mettant en oeuvre une telle composition, le cas échéant en présence d'une composition oxydante.

Elle concerne enfin un dispositif comprenant un premier compartiment renfermant une composition colorante selon l'invention et comprenant un second compartiment renfermant une composition oxydante.

La composition selon l'invention occasionne moins de dégradation des propriétés de coloration et permet d'obtenir des colorations plus puissantes, plus homogènes et plus chromatiques, tout en conférant aux fibres traitées de bonnes propriétés cosmétiques et en limitant leur dégradation.

Les compositions conformes à la présente invention présentent par ailleurs une texture idéale pour un usage en teinture des fibres kératiniques humaines, et en particulier des cheveux. Elles sont en effet onctueuses, suffisamment épaisses pour une application rapide et facile, avec une bonne élimination au rinçage, sans pour autant couler hors des zones de la chevelure que l'on souhaite traiter.

Mais d'autres caractéristiques et avantages de l'invention apparaîtront plus clairement à la lecture de la description et des exemples qui vont suivre.

Dans ce qui va suivre, et à moins d'une indication différente, il est précisé que les bornes de gammes de valeurs sont incluses dans ces gammes.

Lorsqu'il sera fait mention d'un composé à chaîne grasse, cette chaîne est une chaîne hydrocarbonée, saturée ou non, linéaire ou ramifiée, comprenant de 8 à 30 atomes de carbone, de préférence de 10 à 24 atomes de carbone.

De plus la présente invention est appropriée pour la coloration des fibres kératiniques, notamment humaines, telles que les cheveux plus particulièrement.

Ainsi, comme cela a été indiqué précédemment, la composition colorante selon l'invention possède une teneur en eau d'au moins 40 % en poids par rapport au poids de ladite composition colorante.

Selon une variante plus particulière, la teneur en eau dans la composition selon l'invention représente au moins 45 % en poids par rapport au poids de ladite composition colorante.

Conformément à un mode particulièrement préféré de l'invention, la teneur en eau représente au moins 50 % en poids par rapport au poids de ladite composition colorante.

La composition selon l'invention comprend par ailleurs au moins un tensioactif non ionique ou anionique, ou leurs mélanges.

Plus particulièrement, le tensioactif non ionique est choisi parmi :
- les alcools gras oxyalkylénés ou glycérolés ;
- les alkylphénols dont la chaîne alkyle est en C₈-C₁₈, oxyalkylénés ;
- les amides gras oxyalkylénés ou glycérolés ;
- les huiles végétales oxyalkylénées ;
- les esters d'acides C₆-C₃₀ du sorbitan, éventuellement oxyalkylénés ;
- les esters d'acides gras du sucrose, éventuellement oxyalkylénés ;
- les esters d'acides gras du polyéthylèneglycol ;
- les alkyl(C₆-C₃₀)polyglycosides ;
- les dérivés de N-alkyl(C₆-C₃₀)glucamine ;
- les oxydes d'amines tels que les oxydes d'alkyl (C₁₀ - C₁₄) amines ou les oxydes de N-acylaminopropylmorpholine ;
- les copolymères d'oxyde d'éthylène et de propylène ;
- leurs mélanges.

Plus particulièrement, le nombre moyen de motifs oxyalkylénés est avantageusement compris entre 2 et 150 motifs. De préférence, il s'agit de motifs oxyéthylénés, oxypropylénés ou leurs mélanges.

En ce qui concerne les tensioactifs polyglycérolés, ils comportent de préférence en moyenne 1 à 20 groupements glycérol et en particulier 1,5 à 5.

Conformément à un mode de réalisation particulièrement avantageux de l'invention, la composition comprend au moins un tensioactif non ionique choisi parmi les alcools en C₆-C₃₀, oxyalkylénés ou glycérolés.

En ce qui concerne le tensioactif anionique, ce dernier est plus particulièrement choisi parmi :
- les alkyl(C₆-C₃₀)sulfates, les alkyl(C₆-C₃₀)éthersulfates, les alkyl(C₆-C₃₀)amido-éthersulfates, les alkylarylpolyéthersulfates, les monoglycérides sulfates ;
- les alkyl(C₆-C₃₀)sulfonates, les alkyl(C₆-C₃₀)amidesulfonates, les alkyl(C₆-C₃₀)aryl-sulfonates, les α-oléfine-sulfonates, les paraffine-sulfonates ;
- les alkyl(C₆-C₃₀)phosphates ;
- les alkyl(C₆-C₃₀)sulfosuccinates, les alkyl(C₆-C₃₀) éthersulfosuccinates, les alkyl(C₆-C₃₀) amidesulfosuccinates ;
- les alkyl(C₆-C₃₀)sulfoacétates ;
- les acyl(C₆-C₂₄)sarcosinates ;
- les acyl(C₆-C₂₄)glutamates ;
- les éthers d'alkyl(C₆-C₃₀)polyglycosides carboxyliques ; les alkyl(C₆-C₃₀) polyglycoside sulfosuccinates ;
- les alkyl(C₆-C₃₀)sulfosuccinamates ;
- les acyl(C₆-C₂₄)iséthionates ;
- les N-acyl(C₆-C₂₄)taurates ;
- les sels d'acides gras ; les sels d'acides d'huile de coprah ou d'huile de coprah hydrogénée ;
- les acyl(C₈-C₂₀)-lactylates ;
- les sels d'acides d'alkyl(C₆-C₃₀)-D galactoside uroniques ;
- les sels d'acides alkyl(C₆-C₃₀)éther carboxyliques polyoxyalkylénés, d'alkyl(C₆-C₃₀)aryl éther carboxyliques polyoxyalkylénés, d'alkyl(C₆-C₃₀)amido éther carboxyliques polyoxyalkylénés ;
- et leurs mélanges.

Ces tensioactifs anioniques se trouvent avantageusement sous forme de sels dans la composition selon l'invention, notamment de sels de métaux alcalins, comme le sodium ; de métaux alcalino-terreux, comme par exemple le magnésium ; de sels d'ammonium ; de sels d'amines ; de sels d'aminoalcools. Ils pourraient aussi, selon les conditions, se trouver sous leur forme acide.

A noter que les radicaux alkyle ou acyle de ces différents composés comportent de préférence de 12 à 20 atomes de carbone. De préférence, le radical aryle désigne un groupement phényle ou benzyle.

De plus, les tensioactifs anioniques polyoxyalkylénés comportent de préférence de 2 à 50 groupements oxyde d'alkylène en particulier d'éthylène.

Conformément à un mode de réalisation préféré de l'invention, le tensioactif anionique est choisi parmi les sels d'acides gras.

Selon un mode de réalisation particulier de l'invention, la teneur totale en tensioactif non ionique et/ou anionique, représente de 0,01 % à 50 % en poids par rapport au poids de la composition colorante, de préférence de 0,5 à 40 % en poids par rapport au poids de la composition colorante.

Parmi les ingrédients de la composition selon l'invention figure l'hydroxyéthylcellulose.

De préférence, le poids moléculaire de l'hydroxyéthylcellulose entrant dans la composition selon l'invention est d'au moins 700000, et encore plus préférentiellement d'au moins 10⁶.

Le poids moléculaire peut être déterminé à partir de plusieurs techniques dont la chromatographie par perméation de gel et la chromatographie par exclusion de taille.

Parmi les composés appropriés, on peut citer notamment l'hydroxyéthylcellulose de poids moléculaire de 1,3.10⁶, vendue par exemple sous la dénomination commerciale NATROSOL 250 HHR par la Société Aqualon (Hercules), l'hydroxyéthylcellulose de poids moléculaire de 10⁶, vendue sous la dénomination commerciale CELLOSIZE HYDROXYETHYL CELLULOSE PCG-10 par la Société Amerchol, l'hydroxyéthylcellulose de poids moléculaire de 720000, vendue sous la dénomination commerciale NATROSOL 250 MR par la Société Aqualon (Hercules).

Avantageusement, la teneur en hydroxyéthylcellulose représente de 0,01 % à 5 % en poids par rapport au poids de la composition colorante, de préférence de 0,05 à 2,5 % en poids par rapport au poids de la composition colorante. Et plus préférentiellement de 0,1 à 1 % en poids par rapport au poids de la composition colorante

Comme indiqué auparavant, la composition comprend en outre au moins un corps gras.

Avantageusement, le corps gras est choisi parmi les alcools gras non oxyalkylénés et non glycérolés, les amides d'acide gras non oxyalkylénés et non glycérolés, les mono- et poly- esters d'acides carboxyliques, les huiles minérales, les huiles végétales, ou leurs mélanges.

En ce qui concerne les alcools gras, on peut citer les alcools linéaires ou ramifiés, saturés ou insaturés, en C₈-C₃₀, plus particulièrement en C₁₀-C₂₄, de préférence C₁₂-C₂₄, comportant éventuellement au moins un autre groupement hydroxyle. A titre d'exemples, on peut citer entre autres les alcools oléique, laurique, palmitique, myristique, béhénique, stéarique, linoléique, linolénique, caprique, arachidonique, ou leurs mélanges.

Les amides d'acide gras sont plus particulièrement choisis parmi les composés dérivant d'une alcanolamine et d'un acide gras en C₈-C₃₀. De préférence, ils sont choisis parmi les amides d'une alcanolamine en C₂-C₁₀ et d'un acide gras en C₁₄-C₃₀, et encore plus préférentiellement parmi les amides d'une alcanolamine en C₂-C₁₀ et d'un acide gras en C₁₄-C₂₂.

Avantageusement, l'amide d'acide gras est choisi parmi :
- le diéthanolamide d'acide oléique, tel que l'amide commercialisé sous la dénomination commerciale MEXANYL^{®} GT par la société CHIMEX,
- le monoéthanolamide d'acide myristique, tel que l'amide commercialisé sous la dénomination commerciale COMPERLAN^{®} MM par la société COGNIS,
- le diéthanolamide d'acides gras de soja, tel que l'amide commercialisé sous la dénomination commerciale COMPERLAN^{®} VOD par la société COGNIS,
- l'éthanolamide d'acide stéarique, tel que l'amide commercialisé sous la dénomination commerciale MONAMID^{®} S par la société UNIQEMA,
- le monoisopropanolamide d'acide oléique, tel que l'amide commercialisé sous la dénomination commerciale WITCAMIDE^{®} 61 par la société WITCO,
- le diéthanolamide d'acide linoléique, tel que l'amide commercialisé sous la dénomination commerciale PURTON^{®} SFD par la société ZSCHIMMER SCHWARZ,
- le monoéthanolamide d'acide stéarique, tel que l'amide commercialisé sous la dénomination commerciale MONAMID^{®} 972 par la société ICI/UNIQEMA,
- le monoéthanolamide d'acide béhénique, tel que l'amide commercialisée sous la dénomination commerciale INCROMIDE^{®} BEM de CRODA,
- le monoisopropanolamide d'acide isostéarique, tel que l'amide commercialisé sous la dénomination commerciale WITCAMIDE^{®} SPA par la société WITCO,
- le diéthanolamide d'acide érucique, tel que l'amide commercialisé sous la dénomination commerciale diéthanolamide d'acide érucique par la société STEARINERIES DUBOIS,
- le monoéthanolamide d'acide ricinoléique, tel que l'amide commercialisé sous la dénomination commerciale monoéthanolamide ricinoléique par la société STEARINERIES DUBOIS.

En ce qui concerne les mono- ou poly- esters d'acides carboxyliques, ces derniers, linéaires ou ramifiés, saturés ou non, comprennent avantageusement au moins une chaîne hydrocarbonée en C₈-C₃₀, plus particulièrement en C₁₀-C₂₄, de préférence C₁₂-C₂₄, provenant de la partie acide ou alcool, et au moins une chaîne en C₁-C₈, de préférence en C₁-C₆. De plus, si l'acide carboxylique comprend plusieurs fonctions carboxyliques, celles-ci sont de préférence toutes estérifiées. Enfin, il est à noter que les alcools sont de préférence des alcools monofonctionnels.

A titre d'exemples, on peut citer les esters des acides oléique, laurique, palmitique, myristique, béhénique, stéarique, linoléique, linolénique, caprique, arachidonique, ou leurs mélanges comme notamment les mélanges oléo-palmitique, oléo-stéarique, palmito-stéarique, etc.

On peut de plus citer le diester isopropylique de l'acide sébacique (sébaçate de diisopropyle), l'adipate de di-octyle et le maléate de di-caprylyle.

De préférence, les esters sont choisis parmi ceux obtenus à partir d'acides en C₁₂-C₂₄, plus particulièrement comprenant un groupement carboxylique, et d'un monoalcool saturé, linéaire ou ramifié, en C₃-C₆.

Selon cette variante particulièrement avantageuse de l'invention, l'ester est choisi parmi le palmitate d'isopropyle, le myristate d'isopropyle, seuls ou en mélanges.

L'huile de paraffine est un exemple d'huile minérale susceptible d'être utilisée comme corps gras dans la composition.

En ce qui concerne les huiles végétales, on peut citer tout particulièrement l'huile d'avocat, l'huile d'olive ou la cire liquide de jojoba.

De préférence, le corps gras est choisi parmi les alcools gras non oxyalkylénés et non glycérolés.

Selon un mode de réalisation avantageux de l'invention, la teneur totale en corps gras représente au plus 10 % en poids de la composition colorante. De préférence, la teneur totale en corps gras représente de 1 à 9 % en poids par rapport au poids de la composition colorante.

Conformément à l'invention, le rapport pondéral de la quantité totale de tensioactifs non ioniques et anioniques à la quantité de corps gras est supérieur à 1,75, plus particulièrement supérieur à 2 ou de préférence supérieur à 3.

Le rapport pondéral de la quantité totale de tensioactifs non ioniques et anioniques à la quantité totale de corps gras est avantageusement inférieur à 20, plus particulièrement inférieur à 15, et de préférence inférieur à 10.

Comme indiqué auparavant, la composition selon l'invention comprend au moins un polymère associatif cationique.

La structure chimique des polymères associatifs est caractérisée par la présence de zones hydrophiles assurant la solubilité dans l'eau, et de zones hydrophobes par lesquelles les polymères, dans un milieu aqueux, s'assemblent entre eux ou avec les parties hydrophobes d'autres molécules. De tels polymères sont aussi capables, dans un milieu aqueux, de s'associer réversiblement entre eux ou avec d'autres molécules.

Les polymères associatifs mis en oeuvre dans le cadre de l'invention sont plus particulièrement des polymères amphiphiles comportant au moins une chaîne grasse.

Par polymère, on entend au sens de l'invention, des composés présentant dans leur structure la répétition d'au moins un enchaînement autre que l'enchaînement oxyde d'éthylène ou de propylène ou glycérol si ce type d'enchaînement est présent.

De manière avantageuse, le polymère associatif cationique présent dans la composition selon l'invention est choisi parmi les dérivés de cellulose quaternisée, les polyuréthanes cationiques, les polyvinyllactames cationiques et les terpolymères acryliques cationiques ; ces composés comprenant au moins une chaîne grasse.

### Les dérivés de cellulose quaternisée

Ces polymères sont avantageusement choisis parmi :
- les celluloses quaternisées modifiées par des groupements comportant au moins une chaîne grasse, tels que les groupes alkyle, arylalkyle, alkylaryle, de préférence comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant de préférence au moins 8 atomes de carbone, ou des mélanges de ceux-ci.

Les radicaux alkyle portés par les celluloses ou les hydroxyéthylcelluloses quaternisées ci-dessus comportent de préférence de 8 à 30 atomes de carbone. Les radicaux aryle désignent de préférence les groupements phényle, benzyle, naphtyle ou anthryle. On peut indiquer comme exemples d'alkylhydroxyéthylcelluloses quaternisées à chaînes grasses en C₈-C₃₀, les produits QUATRISOFT LM 200, QUATRISOFT LM-X 529-18-A, QUATRISOFT LM-X 529-18B (alkyle en C₁₂) et QUATRISOFT LM-X 529-8 (alkyle en C₁₈) commercialisés par la société AMERCHOL et les produits CRODACEL QM, CRODACEL QL (alkyle en C₁₂) et CRODACEL OS (alkyle en C₁₈) commercialisés par la société CRODA.

### Les polyuréthanes cationiques

Selon une variante particulière de l'invention, les polyuréthanes associatifs cationiques convenables correspondent à la formule générale (la) suivante :

R-X-(P)ₙ-[L-(Y)ₘ]ᵣL'-(P')ₚ-X'-R' (Ia)

dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ; P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ;
la molécule contenant au moins une fonction amine protonée ou quaternisée et au moins un groupement hydrophobe.

Ces composés ont notamment été décrits dans la demande de brevet européen EP1174450.

Dans un mode de réalisation préféré, les seuls groupements hydrophobes sont les groupes R et R' aux extrémités de chaîne.

Une famille préférée de polyuréthanes amphiphiles cationiques est celle correspondant à la formule (la) décrite ci-dessus et dans laquelle :
R et R' représentent tous les deux indépendamment un groupement hydrophobe,
X, X' représentent chacun un groupe L",
n et p valent entre 1 et 1000 et
L, L', L", P, P', Y et m ont la signification indiquée ci-dessus.

Une autre famille préférée de polyuréthanes amphiphiles cationiques est celle correspondant à la formule (la) ci-dessus dans laquelle :
R et R' représentent tous les deux indépendamment un groupement hydrophobe,
X, X' représentent chacun un groupe L", n et p valent 0, et L, L', L", Y et m ont la signification indiquée ci-dessus.

Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthanes amphiphiles cationiques est celle correspondant à la formule (la) ci-dessus dans laquelle :
R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthanes amphiphiles cationiques est comprise de préférence entre 400 et 500 000, en particulier entre 1000 et 400 000 et idéalement entre 1000 et 300 000.

Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel.

A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Lorsque X et/ou X' désignent un groupement comportant une amine tertiaire ou quaternaire, X et/ou X' peuvent représenter l'une des formules suivantes : 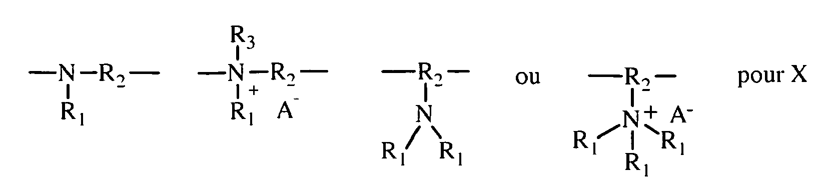 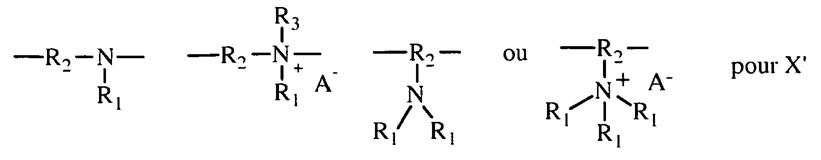 dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A- est un contre-ion physiologiquement acceptable.

Les groupements L, L' et L" représentent un groupe de formule : 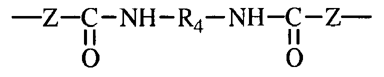 dans laquelle :
Z représente -O-, -S- ou -NH- ; et
R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 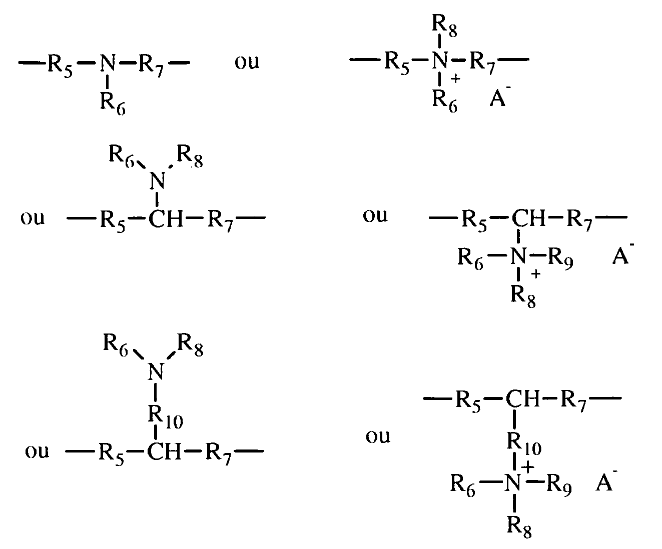 dans lesquelles :
R₅ et R₇ ont les mêmes significations que R₂ défini précédemment;
R₆, R₈ et R₉ ont les mêmes significations que R₁ et R₃ définis précédemment ;
R₁₀ représente un groupe alkylène, linéaire ou ramifié, éventuellement insaturé et pouvant contenir un ou plusieurs hétéroatomes choisis parmi N, O, S et P,
et A⁻ est un contre-ion physiologiquement acceptable.

En ce qui concerne la signification de Y, on entend par groupement hydrophile, un groupement hydrosoluble polymérique ou non.

A titre d'exemple, on peut citer, lorsqu'il ne s'agit pas de polymères, l'éthylèneglycol, le diéthylèneglycol et le propylèneglycol.

Lorsqu'il s'agit, conformément à un mode de réalisation préféré de l'invention, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (la) est facultatif. En effet, les motifs à fonction amine quaternaire ou protonée peuvent suffire à apporter la solubilité ou l'hydrodispersibilité nécessaire pour ce type de polymère dans une solution aqueuse.

Bien que la présence d'un groupe Y hydrophile soit facultative, on préfère cependant des polyuréthanes amphiphiles cationiques comportant un tel groupe.

Lesdits polyuréthanes amphiphiles cationiques sont hydrosolubles ou hydrodispersibles.

### Les polyvinyllactames cationiques

Les polymères poly(vinyllactame) cationiques associatifs utilisables dans le cadre de la présente invention comprennent :
-a) au moins un monomère de type vinyllactame ou alkylvinyllactame;
-b) au moins un monomère de structures (Ib) ou (IIb) suivantes : 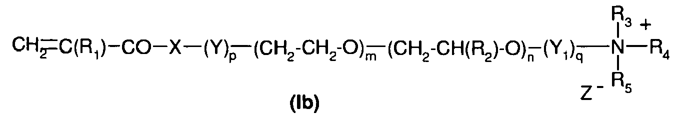 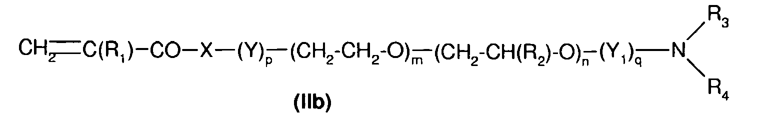
dans lesquelles :
X désigne un atome d'oxygène ou un radical NR₆,
R₁ et R₆ désignent, indépendamment l'un de l'autre, un atome d'hydrogène ou un radical alkyl linéaire ou ramifié en C₁-C₅,
R₂ désigne un radical alkyle linéaire ou ramifié en C₁-C₄,
R₃, R₄ et R₅ désignent, indépendamment l'un de l'autre, un atome d'hydrogène, un radical alkyle linéaire ou ramifié en C₁-C₃₀ ou un radical de formule (IIIb) :

   -(Y₂)ᵣ-(CH₂-CH(R₇)-O)ₓ-R₈ (IIIb)
Y, Y₁ et Y₂ désignent, indépendamment l'un de l'autre, un radical alkylène linéaire ou ramifié en C₂-C₁₆,
R₇ désigne un atome d'hydrogène, ou un radical alkyle linéaire ou ramifié en C₁-C₄ ou un radical hydroxyalkyle linéaire ou ramifié en C₁-C₄,
R₈ désigne un atome d'hydrogène ou un radical alkyle linéaire ou ramifié en C₁-C₃₀,
p, q et r désignent, indépendamment l'un de l'autre, soit la valeur zéro, soit la valeur 1,
m et n désignent, indépendamment l'un de l'autre, un nombre entier allant de 0 à 100,
x désigne un nombre entier allant de 1 à 100,
Z désigne un anion d'acide organique ou minéral,
sous réserve que :
   - l'un au moins des substituants R₃, R₄, R₅ ou R₈ désigne un radical alkyle linéaire ou ramifié en C₈-C₃₀, de préférence en C₉-C₃₀,
   - si m ou n est différent de zéro, alors q est égal à 1,
   - si m ou n sont égaux à zéro, alors p ou q est égal à 0.

Les polymères poly(vinyllactame) cationiques associatifs utilisables selon l'invention peuvent être réticulés ou non réticulés et peuvent aussi être des polymères blocs.

De préférence le contre ion Z⁻ des monomères de formule (Ib) est choisi parmi les ions halogénures, les ions phosphates, l'ion méthosulfate, l'ion tosylate.

De préférence R₃, R₄ et R₅ désignent, indépendamment l'un de l'autre, un atome d'hydrogène ou un radical alkyle linéaire ou ramifié en C₁-C₃₀.

Plus préférentiellement, le monomère b) est un monomère de formule (Ib) pour laquelle, encore plus préférentiellement, m et n sont égaux à zéro.

Le monomère vinyllactame ou alkylvinyllactame est de préférence un composé de structure (IVb) : 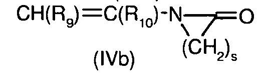 dans laquelle :
s désigne un nombre entier allant de 3 à 6,
R₉ désigne un atome d'hydrogène ou un radical alkyle en C₁-C₅,
R₁₀ désigne un atome d'hydrogène ou un radical alkyle en C₁-C₅,
sous réserve que l'un au moins des radicaux R₉ et R₁₀ désigne un atome d'hydrogène.

Encore plus préférentiellement, le monomère (IVb) est la vinylpyrrolidone.

Les polymères poly(vinyllactame) cationiques peuvent également contenir un ou plusieurs monomères supplémentaires, de préférence cationiques ou non ioniques.

A titre de composés plus particulièrement préférés selon l'invention, on peut citer les terpolymères suivants comprenant au moins :
a)-un monomère de formule (IVb),
b)-un monomère de formule (Ib) dans laquelle p=1, q=0, R₃ et R₄ désignent, indépendamment l'un de l'autre, un atome d'hydrogène ou un radical alkyle en C₁-C₅ et R₅ désigne un radical alkyle en C₈-C₂₄, de préférence en C₉-C₂₄ et
c)-un monomère de formule (IIb) dans laquelle R₃ et R₄ désignent, indépendamment l'un de l'autre, un atome d'hydrogène ou un radical alkyle en C₁-C₅.

Encore plus préférentiellement, on utilisera les terpolymères comprenant, en poids, 40 à 95% de monomère (a), 0,1 à 55% de monomère (c) et 0,25 à 50% de monomère (b). De tels polymères sont décrits dans la demande de brevet WO-00/68282 dont le contenu fait partie intégrante de l'invention.

Comme polymères poly(vinyllactame) cationiques selon l'invention, on utilise notamment les terpolymères vinylpyrrolidone / diméthylaminopropylméthacrylamide / tosylate de dodécyldiméthylméthacrylamidopropylammonium, les terpolymères vinylpyrrolidone /diméthylaminopropylméthacrylamide/ tosylate de cocoyldiméthyl-méthacrylamidopropylammonium, les terpolymères vinylpyrrolidone / diméthylaminopropylméthacrylamide / tosylate ou chlorure de lauryldiméthylméthacrylamidopropyl-ammonium.

La masse moléculaire en poids des polymères poly(vinyllactame) associatifs cationiques selon la présente invention est de préférence comprise entre 500 et 20.10⁶. Elle est plus particulièrement comprise entre 200000 et 2.10⁶, et encore plus préférentiellement comprise entre 400000 et 800000.

### Les terpolymères acryliques

Parmi ces polymères on peut citer les terpolymères acryliques comprenant :
- de 5 à 80% en poids, préférentiellement de 15 à 70% en poids et plus préférentiellement de 40 à 70% en poids, d'un monomère acrylate (a), choisi parmi un acrylate d'alkyle en C₁-C₆ et un méthacrylate d'alkyle en C₁-C₆;
- de 5 à 80% en poids, préférentiellement de 10 à 70% en poids et plus préférentiellement de 20 à 60% en poids, d'un monomère (b), choisi parmi un composé vinylique héterocyclique contenant au moins un atome d'azote ou de soufre, un (méth)acrylamide, un (méth)acrylate de mono- ou di-(C₁-C₄)alkylamino(C₁-C₄)alkyle et un mono ou di- (C₁-C₄)alkylamino(C₁-C₄)alkyl (méth)acrylamide;
- de 0,1 à 30% en poids, préférentiellement de 0,1 à 10% en poids d'un monomère (c), choisi parmi (i) un uréthane produit par réaction entre un isocyanate insaturé monoéthylénique et un tensioactif non ionique à terminaison C₁-C₄ alkoxy; (ii) un copolymère à blocs de 1,2-butylène oxyde et de 1,2-éthylène oxyde; (iii) un monomère tensioactif insaturé éthylénique copolymérisable obtenu par condensation d'un tensioactif non-ionique avec un acide carboxylique insaturé α,β-éthylénique ou son anhydride; (iv) un monomère tensioactif choisi parmi les produits de réaction de type urée d'un monoisocyanate insaturé monoéthylénique avec un tensioactif non-ionique présentant une fonction amine; (v) un éther de (méth)allyle de formule CH₂= CR₁CH₂OAₘBₙAₚR₂ dans lequel R₁ désigne un atome d'hydrogène ou un groupe méthyle, A désigne un groupement propylèneoxy ou butylèneoxy, B désigne l'éthylèneoxy, n est égal à zéro ou désigne un nombre entier inférieur ou égal à 200 et préférentiellement inférieur à 100, m et p désignent zéro ou un nombre entier inférieur à n et R₂ est un groupe hydrophobe d'au moins 8 atomes de carbone et préférentiellement en C₈-C₃₀; et (vi) un monomère non-ionique de type uréthane produit par réaction d'un tensioactif non ionique monohydrique avec un isocyanate insaturé monoéthylénique;les pourcentages en poids de monomères étant basés sur le poids total des monomères constituant le terpolymère.

Des monomères acrylates (a) préférés comprennent notamment les acrylates d'alkyle en C₂-C₆. L'acrylate d'éthyle est tout particulièrement préféré.

Comme exemples de monomères (b) préférés, il faut citer le méthacrylate de N,N-diméthylaminoéthyle (DMAEMA), l'acrylate de N,N-diéthylaminoéthyle, le méthacrylate de N,N-diéthylaminoéthyle, l'acrylate de N-t-butylaminoéthyle, le méthacrylate de N-t-butylaminoéthyle, le N,N-diméthylaminopropyl-acrylamide, le N,N-diméthylaminopropyle-méthacrylamide, le N,N-diéthylaminopropyl-acrylamide et le N,N-diéthylaminopropyl-méthacrylamide. Le méthacrylate de N,N-diméthylaminoéthyle est tout particulièrement préféré.

Les monomères (c) préférés sont les monomères tensioactifs insaturés éthyléniques copolymérisables obtenus par condensation d'un tensioactif non ionique avec un acide carboxylique insaturé α,β-éthylénique ou son anhydride, de préférence les acides mono ou di-carboxyliques en C₃-C₄ ou leurs anhydrides et plus particulièrement l'acide acrylique, l'acide méthacrylique, l'acide crotonique, l'acide maléique, l'anhydride maléique et tout particulièrement l'acide itaconique et l'anhydride itaconique.

Les monomères (c) particulièrement préférés correspondent aux monomères tensioactifs insaturés éthyléniques copolymérisables obtenus par condensation d'un tensioactif non ionique avec l'acide itaconique. Parmi les tensioactifs non ioniques, on peut citer notamment les alcools gras en C₁₀-C₃₀ alkoxylés avec 2 à 100, et de préférence de 5 à 50 moles d'oxyde d'alkylène, comme par exemple les éthers de polyéthylène glycol et d'alcools gras en C₁₀-C₃₀ et plus particulièrement les éthers de polyéthylène glycol et d'alcool cétylique, dénommés CETETH dans le dictionnaire CTFA, 7ème édition, 1997.

Des méthodes conventionnelles pour préparer ces terpolymères acryliques sont connues de l'homme du métier. De telles méthodes incluent la polymérisation en solution, la polymérisation par précipitation et la polymérisation en émulsion. Des terpolymères conformes à l'invention et leurs méthodes de préparation sont notamment décrits dans les demandes EP-A-0824914 et EP-A-0825200.
Parmi ces terpolymères, on préfère utiliser en particulier le polymère "STRUCTURE ^{®} PLUS" vendu par la Société NATIONAL STARCH, qui est constitué d'acrylates, d'amino(méth)acrylates et d'itaconate d'alkyle en C₁₀-C₃₀ polyoxyéthyléné à 20 moles d'oxyde d'éthylène sous forme de dispersion aqueuse à 20% de Matière Active.
En plus de ces monomères, les terpolymères peuvent contenir d'autres monomères qui permettent de réticuler lesdits terpolymères. Ces monomères sont utilisés dans des proportions assez faibles, jusqu'à 2% en poids par rapport au poids total des monomères utilisés pour préparer les terpolymères. De tels monomères de réticulation comprennent des monomères aromatiques portant plusieurs substituants vinyle, des monomères alicycliques portant plusieurs substituants vinyle, des esters bi-fonctionnels d'acide phtalique, des esters bi-fonctionnels d'acide méthacrylique, des esters multifonctionnels d'acide acrylique, le N-méthylène-bis-acrylamide et des monomères aliphatiques portant plusieurs substituants vinyle tels que des diènes, triènes et tétraènes.
Des monomères de réticulation peuvent notamment être des divinyl-benzènes, des trivinylbenzènes, le 1,2,4-trivinylcyclohexène, le 1,5-hexadiène, le 1,5,9-décatriène, le 1,9-décadiène, le 1,5-heptadiène, des di-allyl phthalates, de l'éthylène glycol diméthacrylate, des polyéthylène glycol diméthacrylates, des penta- et tétra-acrylates, des triallyl pentaérythritols, des octa-allyl saccharoses, des cycloparaffines, des cyclooléfines et du N-méthylène-bis-acrylamide.

Dans la présente invention, les polymères associatifs cationiques sont utilisés avantageusement en une quantité pouvant varier de 0,01 à 5% en poids par rapport au poids de la composition colorante. De préférence, cette quantité varie de 0,05 à 2,5 % en poids par rapport au poids de la composition colorante. Conformément à un mode de réalisation encore plus préféré, la teneur en polymère associatif cationique représente de 0,1 à 1 % en poids par rapport au poids de la composition colorante.

La composition selon l'invention comprend en outre au moins un précurseur de colorant d'oxydation et/ou au moins un colorant direct.

Le ou les précurseurs de colorants d'oxydation sont choisis parmi les bases d'oxydation et les coupleurs, ou leurs mélanges.

Les bases d'oxydation sont choisies parmi les bases d'oxydation classiquement utilisées pour la coloration d'oxydation, parmi lesquelles on peut notamment citer les paraphénylènediamines, les bis-phénylalkylènediamines, les para-aminophénols, les ortho-aminophénols et les bases hétérocycliques et leurs sels d'addition avec un acide ou avec un agent alcalin.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylène diamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylène diamine, la N-(β-méthoxyéthyl) paraphénylènediamine et la 4'aminophényl 1-(3-hydroxy)pyrrolidine, et leurs sels d'addition avec un acide ou avec un agent alcalin.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide ou avec un agent alcalin.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylène diamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide ou avec un agent alcalin.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide ou avec un agent alcalin.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide ou avec un agent alcalin.

Parmi les bases hétérocycliques, on peut plus particulièrement citer à titre d'exemple les dérivés pyridiniques, en particulier la 2,3 diamino 6-méthoxypyridine ; les dérivés pyrimidiniques, comme notamment la 2, 4, 5, 6 tétraaminopyrimidine ou encore la 4-hydroxy 2, 5, 6 triaminopyrimidine ; et les dérivés pyrazoliques avec en particulier le 1Nβ hydroxyéthyl 4,5 diaminopyrazole ; et leurs sels d'addition avec un acide ou avec un agent alcalin.

Lorsqu'elles sont utilisées, la ou les bases d'oxydation représentent avantageusement de 0,0005 à 12 % en poids par rapport au poids de la composition colorante, et de préférence de 0,005 à 6 % en poids par rapport au poids de la composition colorante.

La composition peut également comprendre, associé à au moins une base d'oxydation, au moins un coupleur, de façon à modifier ou à enrichir en reflets les nuances obtenues.

Le ou les coupleurs utilisables peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques et leurs sels d'addition avec un acide ou avec un agent alcalin.

Ces coupleurs sont plus particulièrement choisis parmi le 2-méthyl 5-amino phénol, le 5-N-(β-hydroxyéthyl)amino 2-méthyl phénol, le 3-amino phénol, le 2-méthyl 5-amino 6-chloro phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2-amino 3-hydroxy pyridine, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, et leurs sels d'addition avec un acide ou avec un agent alcalin.

Lorsqu'ils sont présents, le ou les coupleurs représentent plus particulièrement de 0,0001 à 15 % en poids, et de préférence de 0,005 à 12 % en poids, par rapport au poids de la composition colorante. Conformément à un mode de réalisation encore plus avantageux, le ou les coupleurs représentent de 0,01 à 10 % en poids par rapport au poids total de la composition colorante.

D'une manière générale, les sels d'addition avec un acide sont notamment choisis parmi les chlorhydrates, les bromhydrates, les sulfates, les citrates, les succinates, les tartrates, les tosylates, les benzènesulfonates, les lactates et les acétates.

Plus particulièrement, le ou les colorants directs sont de nature non ionique, cationique ou anionique.

A titre d'exemples non limitatifs, on peut citer les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane et les colorants naturels, seuls ou en mélanges.

II peut par exemple être choisi parmi les colorants benzéniques nitrés rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyt-4-N-(β-hydroxyéthyt)amino-6-nitro benzène,
- le 1-hydroxy-3-nitro-4-N-(β-hydroxyéthyt)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine,
- le1-amino-3-nitro-6-hydroxybenzène.
- le 1-(β-aminoéthyt)amino-2-nitro-4-(β-hydroxyéthytoxy) benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine,
- le 1-amino-2-nitro-4-hydroxy-5-méthylbenzène.

Le colorant direct peut aussi être choisi parmi les colorants directs benzéniques nitrés jaunes et jaune-verts, on peut par exemple citer les composés choisis parmi :
- le 1-β-hydroxyéthyloxy-3-méthylamino-4-nitrobenzène,
- le 1-méthytamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.

On peut aussi mentionner les colorants directs benzéniques nitrés bleus ou violets, comme par exemple :
- le 1-(β-hydroxyéthy!)am!no-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante : 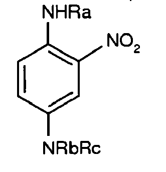
dans laquelle :
- Rb représente un radical alkyle en C₁-C₄, un radical β-hydroxyéthyle ou β-hydroxypropyle ou γ-hydroxypropyle ;
- Ra et Rc, identiques ou différents, représentent un radical β-hydroxyéthyle, -β-hydroxypropyle, γ-hydroxypropyle, ou β,γ-dihydroxypropyle, l'un au moins des radicaux Rb, Rc ou Ra représentant un radical γ-hydroxypropyle et Rb et Rc ne pouvant désigner simultanément un radical β-hydroxyéthyle lorsque Rb est un radical γ-hydroxypropyle, telles que celles décrits dans le brevet français FR 2 692 572.

Parmi les colorants directs azoïques utilisables selon l'invention on peut citer les colorants azoïques cationiques décrits dans les demandes de brevets WO 95/15144, WO 95/01772 et EP 714954, FR 2 822 696, FR 2 825 702, FR 2 825 625, FR 2 822 698, FR 2 822 693, FR 2 822 694, FR 2 829 926, FR 2 807 650, WO 02/078660, WO 02/100834, WO 02/100369, FR 2 844 269.

Parmi ces composés on peut tout particulièrement citer les colorants suivants :
- chlorure de 1,3-diméthyl-2-[[4-(diméthylamino)phényl]azo]-1 H-Imidazolium,
- chlorure de 1 ,3-diméthyl-2-[(4-aminophényl)azo]-1 H-Imidazolium,
- méthylsulfate de 1-méthyl-4-[(méthylphénylhydrazono)méthyl]-pyridinium

On peut également citer parmi les colorants directs azoïques les colorants suivants, décrits dans le COLOUR INDEX INTERNATIONAL 3e édition : Disperse Red 17, Acid Yellow 9, Acid Black 1, Basic Red 22, Basic Red 76, Basic Yellow 57, Basic Brown 16, Acid Yellow 36, Acid Orange 7, Acid Red 33, Acid Red 35, Basic Brown 17, Acid Yellow 23, Acid Orange 24, Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :
Disperse Red 15, Solvent Violet 13, Acid Violet 43, Disperse Violet 1, Disperse Violet 4, Disperse Blue 1, Disperse Violet 8, Disperse Blue 3, Disperse Red 11, Acid Blue 62, Disperse Blue 7, Basic Blue 22, Disperse Violet 15, Basic Blue 99, ainsi que les composés suivants :
   - 1-N-méthylmorpholiniumpropylamino-4-hydroxyanthraquinone
   - 1-Aminopropylamino-4-méthylaminoanthraquinone
   - 1-Aminopropylaminoanthraquinone
   - 5-β-hydroxyéthyl-1,4-diaminoanthraquinone
   - 2-Aminoéthylaminoanthraquinone
   - 1,4-Bis-(β,γ-dihydroxypropylamino)-anthraquinone.

Parmi les colorants aziniques on peut citer les composés suivants :
- Basic Blue 17, Basic Red 2.

Parmi les colorants directs méthiniques cationiques, on peut aussi citer le Basic Red 14, le Basic Yellow 13 et le Basic Yellow 29.

Parmi les colorants triarylméthaniques utilisables selon l'invention, on peut citer les composés suivants : Basic Green 1, Acid blue 9, Basic Violet 3, Basic Violet 14, Basic Blue 7, Acid Violet 49, Basic Blue 26, Acid Blue 7.

Parmi les colorants indoaminiques utilisables selon l'invention, on peut citer les composés suivants :
- 2--hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

La composition peut aussi comprendre des colorants directs naturels comme la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

La teneur en colorants directs, lorsqu'ils sont présents, représente avantageusement de 0,0005 à 15 % en poids par rapport au poids de la composition colorante, et de préférence de 0,005 à 12 % en poids par rapport au poids de la composition colorante. Selon un mode de réalisation encore plus avantageux de l'invention, la teneur en colorants directs, représente de 0,01 à 5 % en poids par rapport au poids de la composition colorante.

La composition selon l'invention peut comprendre en outre au moins un agent alcalinisant.

Parmi les agents alcalinisants on peut citer, à titre d'exemple, l'ammoniaque, les carbonates alcalins, les alcanolamines en C₂-C₁₀ telles que les mono-, di- et triéthanolamines ainsi que leurs dérivés, les hydroxyalkylamines et les ethylènediamines oxyéthylénées et/ou oxypropylénées, les hydroxydes de sodium ou de potassium, les silicates de métaux alcalins ou alcalino-terreux, et les composés de formule suivante : 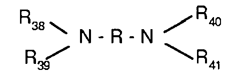 dans laquelle R est un reste propylène éventuellement substitué par un groupement hydroxyle ou un radical alkyle en C₁-C₄ R₃₈, R₃₉, R₄₀ et R₄₁, identiques ou différents, représentent un atome d'hydrogène, un radical alkyle en C₁-C₄ ou hydroxyalkyle en C₁-C₄.

De préférence, l'agent alcalinisant est choisi parmi l'ammoniaque, les alcanolamines, et les associations d'alcanolamines avec des silicates de métaux alcalins ou alcalino-terreux.

Selon un mode de réalisation avantageux de l'invention, la composition ne comprend pas d'ammoniaque en tant qu'agent alcalinisant.

A noter de plus que le pH peut aussi être ajusté en employant des agents acidifiants, comme par exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Plus particulièrement, la teneur en agent alcalinisant et/ou acidifiant est telle que le pH de la composition colorante est compris entre 3 et 12, avantageusement entre 4 et 11 et de préférence entre 7 et 11.

La composition selon l'invention peut aussi comprendre un ou plusieurs polymères substantifs cationiques ou amphotères.

Il est à noter qu'au sens de la présente invention, l'expression "polymère cationique" désigne tout polymère contenant des groupements cationiques et/ou des groupements ionisables en groupements cationiques.

De tels polymères peuvent être choisis parmi ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶, et de préférence comprise entre 10³ et 3.10⁶.

Parmi les polymères cationiques, on peut citer plus particulièrement les polymères du type polyamine, polyaminoamide et polyammonium quaternaire.
Ce sont des produits connus. Ils sont notamment décrits dans les brevets français n°2 505 348 ou 2 542 997. Parmi lesdits polymères, on peut citer :
**(1)** Les homopolymères ou copolymères dérivés d'esters ou d'amides acryliques ou méthacryliques et comportant au moins un des motifs de formules (I), (II), (III) ou (IV) suivantes: 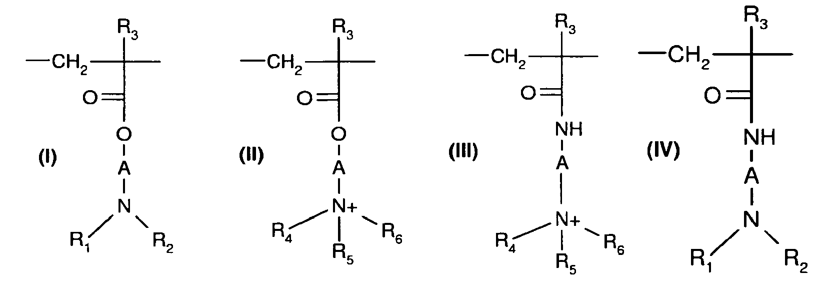 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFOUAT" par la société ISP comme par exemple "GAFOUAT 734" ou "GAFOUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - et les copolymères vinylpyrrolidone / méthacrylamide de diméthylaminopropyle quaternisés tel que le produit vendu sous la dénomination "GAFOUAT HS 100" par la société ISP .
**(2)** Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .
**(3)** Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
**(4)** Les gommes de guar cationiques décrits plus particulièrement dans les brevets US 3589578 et US 4031307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
**(6)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2252840 et 2368508.
**(7)** Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1583363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
**(8)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3227615 et 2961347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
**(9)** Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 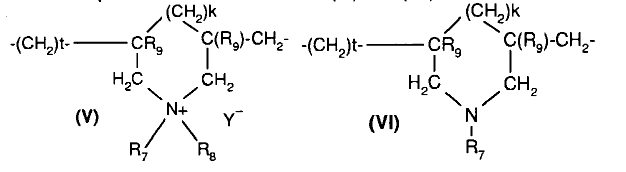 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2080759 et dans son certificat d'addition 2190406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
**(10)** Le polymère de diammonium quaternaire contenant des motifs récurrents répondant à la formule : 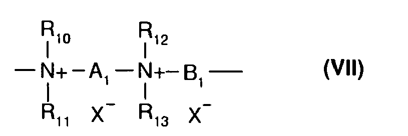 formule (VII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁₀ et R₁₂ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B1 peut également désigner un groupement -(CH₂)ₙ-CO-D-OC-(CH₂)ₙ- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)ₓCH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH-.
   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (VIII) suivante: 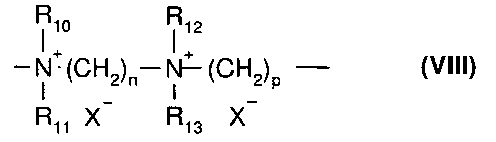 dans laquelle R₁₀, R₁₁, R₁₂ et R₁₃ identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
**(11)** Les polymères de polyammonium quaternaire constitués de motifs récurrents de formule (IX) : 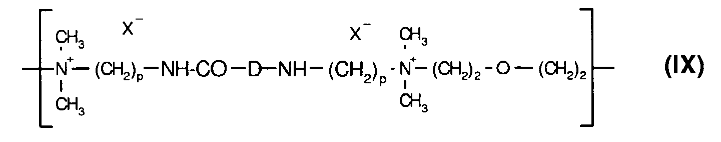 dans laquelle p désigne un nombre entier variant de 1 à 6 environ, D peut être nul ou peut représenter un groupement -(CH₂)ᵣ -CO- dans lequel r désigne un nombre égal à 4 ou à 7, X⁻ est un anion ;
   De tels polymères peuvent être préparés selon les procédés décrits dans les brevets U.S.A. n ° 4 157 388, 4 702 906, 4 719 282. Ils sont notamment décrits dans la demande de brevet EP-A-122 324.
   Parmi eux, on peut par exemple citer, les produits "Mirapol A 15", "Mirapol AD1", "Mirapol AZ1" et "Mirapol 175" vendus par la société Miranol.
**(12)** Les polymères quaternaires de vinylpyrrolidone et de vinylimidazole tels que par exemple les produits commercialisés sous les dénominations Luviquat FC 905, FC 550 et FC 370 par la société B.A.S.F.
**(13)** Les polyamines comme le Polyquart H vendu par HENKEL, référencé sous le nom de " POLYETHYLENEGLYCOL (15) TALLOW POLYAMINE " dans le dictionnaire CTFA.
**(14)** Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de "SALCARE^{®} SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société ALLIED COLLOIDS.
**(15)** D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes : 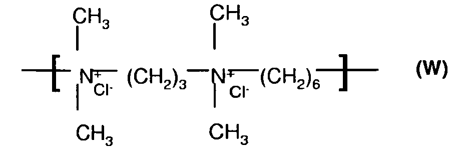 et notamment ceux dont le poids moléculaire, déterminé par chromatographie par perméation de gel, est compris entre 9500 et 9900; 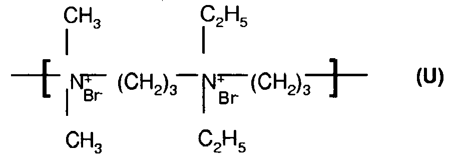 et notamment ceux dont le poids moléculaire, déterminé par chromatographie par perméation de gel, est d'environ 1200.

En ce qui concerne les polymères amphotères utilisables conformément à la présente invention, ceux-ci peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
**(1)** Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.
**(2)** Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
      Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
      Les comonomères basiques préférés sont des méthacrylates d'aminoéthyle, de butyl aminoéthyle, de N,N'-diméthylaminoéthyle, de N-tertio-butylaminoéthyle.
      On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
**(3)** Les polyaminoamides réticulés et alcoylés partiellement ou totalement dérivant de polyaminoamides de formule générale :

   ⁅CO-R₁₉-CO-Z⁆- (X)

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 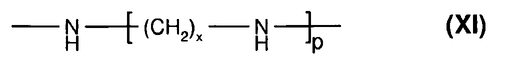 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 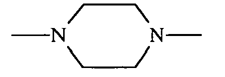
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
      Les alcanes sultones utilisées dans l'alcoylation sont de préférence la propane ou la butane sultone, les sels des agents d'alcoylation sont de préférence les sels de sodium ou de potassium.
**(4)** Les polymères comportant des motifs zwittérioniques de formule : 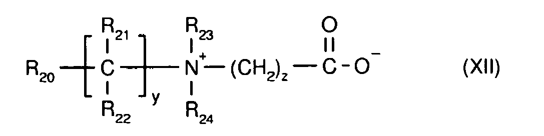 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides
   ou méthacrylamides ou l'acétate de vinyle.
   A titre d'exemple, on peut citer le copolymère de méthacrylate de méthyle / diméthyl-carboxyméthylammonio-éthylméthacrylate de méthyle tel que le produit vendu sous la dénomination DIAFORMER Z301 par la société SANDOZ.
**(5)** Les polymères dérivés du chitosane comportant des motifs monomères répondant aux formules (XIII), (XIV), (XV) suivantes : 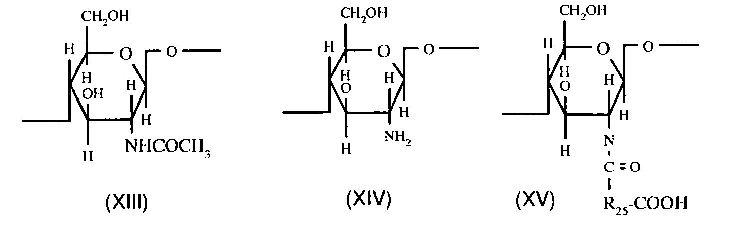 le motif (XIII) étant présent dans des proportions comprises entre 0 et 30%, le motif (XIV) dans des proportions comprises entre 5 et 50% et le motif F dans des proportions comprises entre 30 et 90%, étant entendu que dans ce motif (XV), R₂₅ représente un radical de formule : 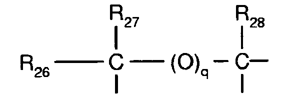 dans laquelle
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
**(6)** Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
**(7)** Les polymères répondant à la formule générale (XVI) tels que ceux décrits par exemple dans le brevet français 1400366 : 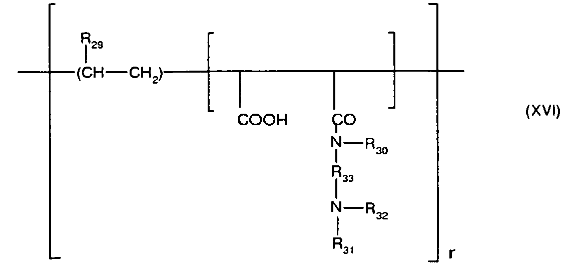 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁ désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂- , -CH₂-CH₂-CH₂- , -CH₂-CH(CH₃)- , R₃₁ ayant les significations mentionnées ci-dessus,
   ainsi que les homologues supérieurs de ces radicaux et contenant jusqu'à 6 atomes de carbone.
**(8)** Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- **(XVII)**

      où D désigne un radical 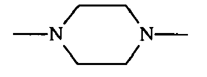 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- **(XVIII)**
   où D désigne un radical 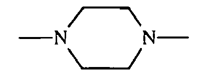 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
**(9)** Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères substantifs cationiques ou amphotères, lorsqu'ils sont présents, représentent avantageusement de 0,01 % à 10 % en poids par rapport au poids de la composition colorante, plus particulièrement de 0,05 % à 5 % en poids par rapport au poids de la composition colorante, et de préférence de 0,1 % à 3 % en poids par rapport au poids de la composition colorante.

Le milieu approprié pour la teinture des fibres kératiniques est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions comprises entre 1 et 40 % en poids par rapport au poids de la composition colorante, et de préférence entre 5 et 30 % en poids par rapport au poids de la composition colorante.

La composition peut encore comprendre des additifs usuels dans le domaine comme notamment des agents épaississants organiques différents de l'hydroxyéthylcellulose décrite précédemment ou minéraux ; des agents antioxydants ; des agents de pénétration ; des agents séquestrants ; des parfums ; des tampons ; des agents dispersants ; des agents de conditionnement différents des polymères substantifs cationiques ou amphotères tels que par exemple des cations, des silicones volatiles ou non volatiles, modifiées ou non modifiées ; des agents filmogènes ; des céramides ; des agents conservateurs ; des agents stabilisants ; des agents opacifiants.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

Comme indiqué auparavant, la présente invention concerne de plus un procédé de coloration de matières kératiniques mettant en oeuvre la composition selon l'invention.

Selon une première variante, le procédé consiste à appliquer la composition en l'absence d'un agent oxydant, sur les matières kératiniques, de préférence des fibres, sèches ou humides, avec ou sans rinçage final de la composition.

Dans le cas de cette variante, la composition selon l'invention ne comprend pas de précurseur de colorant d'oxydation, mais seulement un ou plusieurs colorants directs.

Selon une deuxième variante de l'invention, le procédé consiste à appliquer la composition selon l'invention, en présence d'un agent oxydant, sur les matières kératiniques, sèches ou humides, puis à laisser poser pendant une durée suffisante pour obtenir la coloration souhaitée.

Selon une première possibilité, on applique sur lesdites fibres kératiniques, une ou plusieurs compositions colorantes selon l'invention et une composition oxydante simultanément ou successivement sans rinçage intermédiaire.

De préférence, la composition appliquée est une composition dite prête à l'emploi, c'est-à-dire une composition obtenue par mélange extemporané d'une ou de plusieurs compositions colorantes selon l'invention, avec une composition comprenant au moins un agent oxydant.

Dans ce cas, la composition colorante peut comprendre un ou plusieurs précurseurs de colorants d'oxydation. Elle peut aussi comprendre un ou plusieurs colorants directs, dans le cas où un éclaircissement des fibres kératiniques est souhaité en association avec la coloration.

Bien évidemment la composition colorante peut comprendre une association de précurseurs de colorants d'oxydation et de colorants directs.

L'agent oxydant présents dans la composition oxydante peut être choisi par exemple parmi le peroxyde d'hydrogène, le peroxyde d'urée, les bromates de métaux alcalins, les persels tels que les perborates et persulfates, et les enzymes telles que les peroxydases et les oxydo-réductases à deux ou à quatre électrons. L'utilisation du peroxyde d'hydrogène est particulièrement préférée.

La teneur en agent oxydant est en général comprise entre 1 et 40 % en poids, par rapport au poids de la composition prête à l'emploi de préférence entre 1 et 20 % en poids par rapport au poids de la composition prête à l'emploi.

Généralement, la composition oxydante utilisée est une composition aqueuse et peut se trouver sous la forme d'une solution ou encore d'une émulsion.

Habituellement, on mélange la composition exempte d'agent oxydant avec environ 0,5 à 10 équivalents en poids de la composition oxydante.

Notons que le pH de la composition prête à l'emploi est plus particulièrement compris entre 3 et 12, de préférence entre 4 et 11, et encore plus précisément entre 6,5 et 10,5.

Le pH de la composition prête à l'emploi peut être ajusté au moyen d'un agent alcalinisant ou acidifiant notamment choisi parmi ceux mentionnés auparavant.

Toujours dans le cas où la composition est appliquée en présence d'un agent oxydant, le procédé peut comprendre une étape préliminaire consistant à stocker sous forme séparée, d'une part, une ou plusieurs compositions colorantes selon l'invention et d'autre part, une composition comprenant, dans un milieu approprié pour la teinture des fibres kératiniques humaines, au moins un agent oxydant, puis à procéder à leur mélange au moment de l'emploi avant d'appliquer ce mélange sur les matières kératiniques, sèches ou humides.

Quelle que soit la variante retenue, c'est-à-dire en présence ou en l'absence d'agent oxydant, le temps nécessaire au développement de la coloration est d'environ quelques secondes à 60 minutes et plus particulièrement d'environ 1 à 50 minutes.

La température nécessaire au développement de la coloration est généralement comprise entre la température ambiante (15 à 25°C) et 250°C, plus particulièrement entre la température ambiante et 180°C, de préférence entre la température ambiante et 60°C.

Une fois le temps nécessaire au développement de la coloration écoulé, on élimine de préférence la composition.

Ceci peut avoir lieu de manière classique, soit en mettant en oeuvre au moins un rinçage, soit en mettant en oeuvre un ou plusieurs lavages et rinçage(s), soit en mettant en oeuvre leur combinaison. Enfin, on sèche les matières kératiniques ou on les laisse sécher.

Les exemples suivants illustrent l'invention sans toutefois en limiter la portée.

### EXEMPLE 1

On a préparé la composition colorante alcaline A suivante (quantités exprimées en grammes) :

| | **Quantités (g%)** |
|---|---|
| Acide oléique | 2 |
| Monoéthanolamine pure | 0,47 |
| Alcool oléocétylique oxyéthyléné (30 OE) | 3,15 |
| Alcool laurique oxyéthyléné (12 OE) | 4,4 |
| Alcool décylique oxyéthyléné (5 OE) | 3,15 |
| Alcool décylique oxyéthyléné (3 OE) | 12 |
| Alcool oléique | 1,8 |
| Monoisopropanolamide d'acides de coprah | 4 |
| Hydroxyéthylcellulose (PM : 1300000 ; NATROSOL 250 HHR ; Société Aqualon (Hercules)) | 0,2 |
| Hydroxyéthylcellulose quaternisée par l'époxyde de lauryl diméthyl ammonium substitué (POLYQUATERNIUM-24 ; QUATRISOFT LM 200 ; Société AMERCHOL) | 0,15 |
| Glycérol | 3 |
| Chlorure de polydiméthyl diallyl ammonium dans l'eau à 40 % (Polyquaternium-6) | 3 |
| Acide ascorbique | 0,25 |
| EDTA | 0,2 |
| Thiolactate d'ammonium en solution aqueuse à 58 % | 0,8 |
| Ammoniaque (20% en ammoniac) | 8 |
| 1-hydroxy-4-amino-benzène | 0,545 |
| 1-méthyl-2-hydroxy-4-amino-benzène | 0,615 |
| Parfum | 0,95 |
| eau désionisée | 51,32 |

La composition colorante A est stable au stockage.

La composition colorante A est mélangée, au moment de l'emploi, dans un bol en plastique et pendant 2 minutes, à une composition aqueuse oxydante à 6 % de peroxyde d'hydrogène, à raison de 1 partie de composition colorante pour 1,5 parties de composition oxydante. Le mélange est facile et rapide.

On a appliqué le mélange obtenu sur des mèches de cheveux naturels à 90% de blancs et on a laissé pauser 20 minutes. L'application est facile et rapide. Le produit se localise parfaitement, ne coule pas, et s'étale bien de la racine à la pointe.

On a ensuite rincé les mèches à l'eau, on les a lavées au shampooing standard, à nouveau rincées à l'eau, puis séchées et démêlées. Le mélange s'élimine bien au rinçage.

Les cheveux ont été teints dans une nuance cuivré rouge puissante.

De plus, les cheveux ne sont pas rêches.

### EXEMPLE 2

On a préparé les compositions colorantes alcalines suivantes (quantités exprimées en grammes) :

| | **Composition B invention** | **Composition C comparative** |
|---|---|---|
| Acide oléique | 2 | 2 |
| Monoéthanolamine pure | 0,47 | 0,47 |
| Alcool oléocétylique oxyéthyléné (30 OE) | 3,15 | 4,5 |
| Alcool laurique oxyéthyléné (12 OE) | 4,4 | 6,3 |
| Alcool décylique oxyéthyléné (5 OE) | 3,15 | 4,5 |
| Alcool décylique oxyéthyléné (3 OE) | 12 | 17,2 |
| Alcool oléique | 1,8 | 1,8 |
| Monoisopropanolamide d'acides de coprah | 4 | 4 |
| Hydroxyéthylcellulose (PM : 1300000 ; Natrosol 250 HHR ; Société Aqualon (Hercules)) | 0,2 | / |
| Hydroxyéthylcellulose quaternisée par l'époxyde de lauryldiméthylammonium substitué (Polyquaternium-24 ; Quatrisoft LM 200 ; Société Amerchol) | 0,15 | 0,15 |
| Glycérol | 3 | 3 |
| Polycondensat tetraméthyl hexaméthylène diamine / dichloro 1,3-propylène en solution aqueuse à 60% | 2 | 2 |
| Chlorure de poly diméthyl diallylammonium dans l'eau a 40 % non stabilisé (Polyquaternium-6) | 2 | 2 |
| Acide ascorbique | 0,25 | 0,25 |
| EDTA | 0,2 | 0,2 |
| Thiolactate d'ammonium en solution aqueuse à 58 % | 0,8 | 0,8 |
| Ammoniaque (20% en ammoniac) | 8 | 8 |
| 1-hydroxy-4-amino-benzène | 0,545 | 0,545 |
| 1-méthyl-2-hydroxy-4-amino-benzène | 0,615 | 0,615 |
| Parfum | 0,95 | 0,95 |
| Eau désionisée | 50,32 | 40,72 |

Les compositions colorantes B et C sont stables au stockage.

Les compositions colorantes B et C sont mélangées, au moment de l'emploi, dans un bol en plastique et pendant 2 minutes, à une composition aqueuse oxydante à 6 % de peroxyde d'hydrogène, à raison de 1 partie de composition colorante pour 1,5 parties de composition oxydante. Le mélange est facile et rapide, tout particulièrement pour la composition B.

On a appliqué chaque mélange obtenu sur des mèches de cheveux naturels à 90% de blancs et on a laissé pauser 20 minutes.

On remarque que la composition B se localise parfaitement, ne coule pas, et s'étale mieux de la racine à la pointe, par rapport à la composition C.

On a ensuite rincé chaque mèche à l'eau, on les a lavées au shampooing standard, à nouveau rincées à l'eau, puis séchées et démêlées.

Les mélanges s'éliminent bien au rinçage.

Dans le cas des deux compositions, les cheveux ont été teints dans une nuance cuivré rouge puissante.

La nuance est légèrement plus intense, et moins sélective avec la composition B selon l'invention.

De plus, les cheveux sont moins rêches avec le mélange issu de la composition B selon l'invention.

### EXEMPLE 3

On a préparé la composition colorante sans ammoniaque D suivante (quantités exprimées en grammes) :

| | **Composition D** |
|---|---|
| Acide oléique | 2 |
| Monoéthanolamine pure | 4,72 |
| Alcool oléocétylique (30 OE) | 3,15 |
| Alcool laurique oxyéthyléné (12 OE) | 4,4 |
| alcool décylique oxyéthyléné (5 OE) | 3,15 |
| alcool décylique oxyéthyléné (3 OE) | 12 |
| Alcool oléique | 1,8 |
| Monoisopropanolamide d'acides de coprah | 2 |
| Hydroxyéthylcellulose (PM : 1 300 000) vendue sous la dénomination commerciale Natrosol 250 HHR; société Aqualon | 0,3 |
| Hydroxyéthylcellulose quaternisée par l'époxyde de lauryl | 0,2 |
| diméthyl ammonium substitué (Polyquaternium-24) vendue sous la dénomination commerciale Quatrisoft LM 200 ; société Amerchol | |
| Glycérol | 3 |
| Polycondensat tétraméthyl Hexaméthylènediamine / dichloro 1,3-propylène en solution aqueuse à 60% | 3 |
| Chlorure de polydiméthyl diallyl ammonium dans l'eau a 40 % non stabilisé (Polyquaternium-6) | 3 |
| Acide ascorbique | 0,25 |
| EDTA | 0,2 |
| Métabisulfite de sodium | 0,46 |
| Métasilicate de sodium anhydre | 2 |
| 1-hydroxy-4-amino-benzene | 0,545 |
| 1-methyl-2-hydroxy-4-amino-benzene | 0,615 |
| Parfum | 0,95 |
| Eau désionisée | 52,26 |

La composition colorante D est mélangée, au moment de l'emploi, dans un bol en plastique et pendant 2 minutes, à une composition aqueuse oxydante à 6 % en eau oxygénée, à raison de 1 partie de composition colorante pour 1,5 parties de composition oxydante. Le mélange est facile et rapide.

On a appliqué le mélange obtenu sur des mèches de cheveux naturels à 90% de blancs et on a laissé pauser 20 minutes. L'application est facile et rapide. Le produit se localise parfaitement, ne coule pas, et s'étale bien de la racine à la pointe.

On a ensuite rincé les mèches à l'eau, on les a lavées au shampooing standard, à nouveau rincées à l'eau, puis séchées et démêlées. Le mélange s'élimine bien au rinçage.

Les cheveux ont été teints dans une nuance cuivré rouge puissante. De plus, les cheveux ne sont pas rêches.

### EXEMPLE 4

On a préparé enfin la composition colorante sans ammoniaque E suivante (quantités exprimées en grammes) :

| | **Composition E** |
|---|---|
| Acide oléique | 2 |
| Monoéthanolamine pure | 10,47 |
| Alcool oléocétylique oxyéthyléné (30 OE) | 3,15 |
| Alcool laurique oxyéthyléné (12 OE) | 4,4 |
| Alcool décylique oxyéthyléné (5 OE) | 3,15 |
| Alcool décylique oxyéthyléné (3 OE) | 12 |
| Alcool oléique | 1,8 |
| Monoisopropanolamide d'acides de coprah | 2 |
| Hydroxyéthylcellulose (PM : 1 300 000) vendue sous la dénomination commerciale Natrosol 250 HHR ; société Aqualon | 0,3 |
| Hydroxyéthylcellulose quaternisée par l'époxyde de lauryl di-méthyl ammonium substitué (Polyquaternium-24) vendue sous la dénomination commerciale Quatrisoft LM 200 ; société Amerchol | 0,2 |
| Glycérol | 3 |
| Polycondensat tétraméthyl hexaméthylènediamine / dichloro 1,3-propylène en solution aqueuse à 60% | 3 |
| Chlorure de polydiméthyl diallyl ammonium dans l'eau à 40 % non stabilisé (Polyquaternium-6) | 3 |
| Acide ascorbique | 0,25 |
| EDTA | 0,2 |
| Metabisulfite de sodium | 0,46 |
| 1-hydroxy-4-amino-benzène | 0,545 |
| 1-méthyl-2-hydroxy-4-amino-benzène | 0,615 |
| Parfum | 0,95 |
| Eau désionisée | 48,51 |

La composition colorante E est mélangée, au moment de l'emploi, dans un bol en plastique et pendant 2 minutes, à une composition aqueuse oxydante à 6 % en eau oxygénée, à raison de 1 partie de composition colorante pour 1,5 parties de composition oxydante. Le mélange est facile et rapide.

On a appliqué le mélange obtenu sur des mèches de cheveux naturels à 90% de blancs et on a laissé pauser 20 minutes. L'application est facile et rapide. Le produit se localise parfaitement, ne coule pas, et s'étale bien de la racine à la pointe.

On a ensuite rincé les mèches à l'eau, on les a lavées au shampooing standard, à nouveau rincées à l'eau, puis séchées et démêlées. Le mélange s'élimine bien au rinçage.

Les cheveux ont été teints dans une nuance cuivré rouge puissante. De plus, les cheveux ne sont pas rêches.

## Revendications

1. Composition colorante comprenant, dans un milieu approprié pour la teinture des fibres kératiniques :
• au moins un précurseur de colorant d'oxydation et/ou au moins un colorant direct ;
• au moins un tensioactif non ionique, anionique ou leurs mélanges ;
• au moins l'hydroxyéthylcellulose ;
• au moins un polymère associatif cationique ;
• au moins un corps gras ;
• le rapport pondéral de la quantité totale de tensioactifs non ioniques et anioniques à la quantité totale en corps gras étant supérieure ou égale à 1,75 ; et
• la teneur en eau dans la composition colorante représentant au moins 40 % en poids par rapport au poids de ladite composition colorante.

2. Composition selon la revendication précédente, **caractérisée en ce que** la teneur en eau représente au moins 45 % en poids par rapport au poids de ladite composition colorante.

3. Composition selon l'une quelconque des revendications précédentes, **caractérisée en ce que** la teneur en eau représente au moins 50 % en poids par rapport au poids de ladite composition colorante.

4. Composition selon l'une quelconque des revendications précédentes, **caractérisée en ce que** le tensioactif non ionique est choisi parmi :
• les alcools gras oxyalkylénés ou glycérolés ;
• les alkylphénols dont la chaîne alkyle est en C₈-C₁₈, oxyalkylénés ;
• les amides gras oxyalkylénés ou glycérolés ;
• les huiles végétales oxyalkylénées ;
• les esters d'acides gras du sorbitan, éventuellement oxyalkylénés ;
• les esters d'acides gras du sucrose, éventuellement oxyalkylénés ;
• les esters d'acides gras du polyéthylèneglycol ;
• les alkyl(C₆-C₃₀)polyglycosides ;
• les dérivés de N-alkyl(C₆-C₃₀)glucamine ;
• les oxydes d'amines tels que les oxydes d'alkyl (C₁₀ - C₁₄) amines ou les oxydes de N-acylaminopropylmorpholine ;
• les copolymères d'oxyde d'éthylène et de propylène ;
• leurs mélanges.

5. Composition selon l'une quelconque des revendications précédentes, **caractérisée en ce que** le tensioactif non ionique est choisi parmi les alcools gras, oxyalkylénés ou glycérolés.

6. Composition selon l'une quelconque des revendications précédentes, **caractérisée en ce que** le tensioactif anionique est choisi parmi :
• les alkyl(C₆-C₃₀)sulfates, les alkyl(C₆-C₃₀)éthersulfates, les alkyl(C₆-C₃₀)amido-éthersulfates, les alkylarylpolyéthersulfates, les monoglycérides sulfates ;
• les alkyl(C₆-C₃₀)sulfonates, les alkyl(C₆-C₃₀)amidesulfonates, les alkyl(C₆-C₃₀)aryl-sulfonates, les α-oléfine-sulfonates, les paraffine-sulfonates ;
• alkyl(C₆-C₃₀)phosphates ;
• les alkyl(C₆-C₃₀)sulfosuccinates, les alkyl(C₆-C₃₀) éthersulfosuccinates, les alkyl(C₆-C₃₀) amidesulfosuccinates ;
• les alkyl(C₆-C₃₀)sulfoacétates ;
• les acyl(C₆-C₂₄)sarcosinates ;
• les acyl(C₆-C₂₄)glutamates ;
• les éthers d'alkyl(C₆-C₃₀)polyglycosides carboxyliques ; les alkyl(C₆-C₃₀) polyglycoside sulfosuccinates ;
• les alkyl(C₆-C₃₀)sulfosuccinamates ;
• les acyl(C₆-C₂₄)iséthionates ;
• les N-acyl(C₆-C₂₄)taurates ;
• les sels d'acides gras en C₆-C₃₀ ; les sels d'acides d'huile de coprah ou d'huile de coprah hydrogénée ;
• les acyl(C₈-C₂₀)-lactylates ;
• les sels d'acides d'alkyl(C₆-C₃₀)-D galactoside uroniques ;
• les sels d'acides alkyl(C₆-C₃₀)éther carboxyliques polyoxyalkylénés, d'alkyl(C₆-C₃₀)aryl éther carboxyliques polyoxyalkylénés, d'alkyl(C₆-C₃₀)amido éther carboxyliques polyoxyalkylénés ;
• et leurs mélanges.

7. Composition selon l'une quelconque des revendications précédentes, **caractérisée en ce que** la teneur totale en tensioactif non ionique et/ou anionique représente de 0,01 % à 50 % en poids par rapport au poids de la composition colorante.

8. Composition selon l'une quelconque des revendications précédentes, **caractérisée en ce que** le corps gras est choisi parmi les alcools gras non oxyalkylénés non glycérolés, les amides d'acide gras non oxyalkylénés non glycérolés, les mono- et poly- esters d'acides carboxyliques, les huiles minérales, les huiles végétales, ou leurs mélanges.

9. Composition selon l'une quelconque des revendications précédentes, **caractérisée en ce que** la teneur en corps gras représente au plus 10 % en poids par rapport au poids de la composition colorante.

10. Composition selon l'une quelconque des revendications précédentes, **caractérisée en ce que** le rapport pondéral de la quantité totale de tensioactifs non ioniques et anioniques à la quantité de corps gras est supérieure à 2.

11. Composition selon l'une quelconque des revendications précédentes, **caractérisée en ce que** le rapport pondéral de la quantité totale de tensioactifs non ioniques et anioniques à la quantité de corps gras est inférieur à 20.

12. Composition selon l'une quelconque des revendications précédentes, **caractérisée en ce que** l'hydroxyéthylcellulose de poids moléculaire moyen d'au moins 700000.

13. Composition selon l'une quelconque des revendications précédentes, **caractérisée en ce que** la teneur en hydroxyéthylcellulose représente de 0,01% à 5 % en poids par rapport au poids de la composition colorante.

14. Composition selon l'une quelconque des revendications précédentes, **caractérisée en ce que** le polymère associatif cationique est choisi parmi les dérivés de cellulose quaternisée, les polyuréthanes cationiques, les polyvinyllactames cationiques et les terpolymères acryliques cationiques.

15. Composition selon l'une quelconque des revendications précédentes, **caractérisée en ce que** la teneur en polymère associatif cationique représente de 0,01 à 5% en poids par rapport au poids de la composition colorante.

16. Composition selon l'une quelconque des revendications précédentes, **caractérisée en ce que** le précurseur de colorant d'oxydation est choisi parmi les bases d'oxydation, les coupleurs ou leurs mélanges.

17. Composition selon la revendication 12, **caractérisée en ce que** la teneur en base d'oxydation représente de 0,0005 à 12 % en poids par rapport au poids de la composition colorante.

18. Composition selon la revendication 12, **caractérisée en ce que** la teneur en coupleur représente de 0,0001 à 15 % en poids par rapport au poids de la composition colorante.

19. Composition selon l'une quelconque des revendications précédentes, **caractérisée en ce que** la teneur en colorant direct représente de 0,0005 à 15 % en poids par rapport au poids de la composition colorante.

20. Composition selon l'une quelconque des revendications précédentes, **caractérisée en ce que** la composition comprend au moins un agent alcalinisant.

21. Composition selon la revendication précédente, **caractérisée en ce que** l'agent alcalinisant est choisi parmi l'ammoniaque, les alcanolamines, et les associations d'alcanolamines en C₂-C₁₀ avec des silicates de métaux alcalins ou alcalino-terreux.

22. Composition selon l'une quelconque des revendications précédentes, **caractérisée en ce qu'**elle comprend au moins un polymère substantif cationique ou amphotère.

23. Composition selon la revendication précédente, **caractérisée en ce que** a teneur en polymère substantif cationique ou amphotère représente de 0,01 % à 10 % en poids par rapport au poids de la composition colorante.

24. Composition selon l'une quelconque des revendications précédentes **caractérisée en ce qu'**elle comprend au moins un agent oxydant.

25. Procédé de coloration de fibres kératiniques, dans lequel on applique sur lesdites fibres, sèches ou humides, une composition colorante selon l'une quelconque des revendications 1 à 24.

26. Procédé de coloration de fibres kératiniques dans lequel on applique sur lesdites fibres, sèches ou humides, une composition colorante selon l'une quelconque des revendications 1 à 23, en présence d'une composition oxydante, appliquée simultanément ou successivement sans rinçage intermédiaire à la composition colorante, on laisse poser et on rince les fibres.

27. Procédé de coloration de fibres kératiniques dans lequel on applique sur lesdites fibres, sèches ou humides, une composition colorante selon l'une quelconque des revendications 1 à 23, en présence d'une composition oxydante, mélangée à la composition colorante avant l'application, on laisse poser et on rince les fibres.

28. Dispositif à plusieurs compartiments utilisable pour la coloration de fibres kératiniques, comprenant un premier compartiment renfermant une composition selon l'une quelconque des revendications 1 à 23 et comprenant un second compartiment renfermant une composition oxydante.

## Claims

1. Dye composition comprising, in a medium that is suitable for dyeing keratin fibres:
• at least one oxidation dye precursor and/or at least one direct dye;
• at least one nonionic or anionic surfactant or mixtures thereof;
• at least hydroxyethylcellulose;
• at least one cationic associative polymer;
• at least one fatty substance;
• the weight ratio of the total amount of nonionic and anionic surfactants to the total amount of fatty substances being greater than or equal to 1.75; and
• the water content in the dye composition representing at least 40% by weight relative to the weight of the said dye composition.

2. Composition according to the preceding claim, **characterized in that** the water content represents at least 45% by weight relative to the weight of the said dye composition.

3. Composition according to either of the preceding claims, **characterized in that** the water content represents at least 50% by weight relative to the weight of the said dye composition.

4. Composition according to any one of the preceding claims, **characterized in that** the nonionic surfactant is chosen from:
• oxyalkylenated or glycerolated fatty alcohols;
• oxyalkylenated alkylphenols in which the alkyl chain is of C₈-C₁₈;
• oxyalkylenated or glycerolated fatty amides;
• oxyalkylenated plant oils;
• optionally oxyalkylenated fatty acid esters of sorbitan;
• optionally oxyalkylenated fatty acid esters of sucrose;
• fatty acid esters of polyethylene glycol;
• (C₆-C₃₀)alkylpolyglycosides;
• N-(C₆-C₃₀)alkylglucamine derivatives;
• amine oxides such as (C₁₀-C₁₄)alkylamine oxides or N-acylaminopropylmorpholine oxides;
• copolymers of ethylene oxide and of propylene oxide;
• mixtures thereof.

5. Composition according to any one of the preceding claims, **characterized in that** the nonionic surfactant is chosen from oxyalkylenated or glycerolated fatty alcohols.

6. Composition according to any one of the preceding claims, **characterized in that** the anionic surfactant is chosen from:
• (C₆-C₃₀)alkyl sulfates, (C₆-C₃₀)alkyl ether sulfates, (C₆-C₃₀)alkylamido ether sulfates, alkylaryl polyether sulfates, monoglyceride sulfates;
• (C₆-C₃₀)alkylsulfonates, (C₆-C₃₀)alkylamide sulfonates, (C₆-C₃₀)alkylaryl sulfonates, α-olefin sulfonates, paraffin sulfonates;
• (C₆-C₃₀)alkyl phosphates;
• (C₆-C₃₀)alkyl sulfosuccinates, (C₆-C₃₀)alkyl ether sulfosuccinates, (C₆-C₃₀)alkylamide sulfosuccinates;
• (C₆-C₃₀)alkyl sulfoacetates;
• (C₆-C₂₄)acyl sarcosinates;
• (C₆-C₂₄)acyl glutamates;
• (C₆-C₃₀)alkylpolyglycoside carboxylic ethers; (C₆-C₃₀)alkylpolyglycoside sulfosuccinates;
• (C₆-C₃₀)alkyl sulfosuccinamates;
• (C₆-C₂₄)acyl isethionates;
• N-(C₆-C₂₄)acyl taurates;
• C₆-C₃₀ fatty acid salts; coconut oil acid salts or hydrogenated coconut oil acid salts;
• (C₈-C₂₀)acyl lactylates;
• (C₆-C₃₀)alkyl-D-galactoside uronic acid salts;
• polyoxyalkylenated (C₆-C₃₀)alkyl ether carboxylic acid salts, polyoxyalkylenated (C₆-C₃₀)alkylaryl ether carboxylic acid salts, polyoxyalkylenated (C₆-C₃₀)alkylamido ether carboxylic acid salts;
• and mixtures thereof.

7. Composition according to any one of the preceding claims, **characterized in that** the total content of nonionic and/or anionic surfactant represents from 0.01% to 50% by weight relative to the weight of the dye composition.

8. Composition according to any one of the preceding claims, **characterized in that** the fatty substance is chosen from non-oxyalkylenated, non-glycerolated fatty alcohols, non-oxyalkylenated, non-glycerolated fatty acid amides, carboxylic acid monoesters and polyesters, mineral oils and plant oils, or mixtures thereof.

9. Composition according to any one of the preceding claims, **characterized in that** the content of fatty substance represents not more than 10% by weight relative to the weight of the dye composition.

10. Composition according to any one of the preceding claims, **characterized in that** the weight ratio of the total amount of nonionic and anionic surfactants to the amount of fatty substance is greater than 2.

11. Composition according to any one of the preceding claims, **characterized in that** the weight ratio of the total amount of nonionic and anionic surfactants to the amount of fatty substance is less than 20.

12. Composition according to any one of the preceding claims, **characterized in that** the hydroxyethylcellulose has an average molecular weight of at least 700 000.

13. Composition according to any one of the preceding claims, **characterized in that** the hydroxyethylcellulose content represents from 0.01% to 5% by weight relative to the weight of the dye composition.

14. Composition according to any one of the preceding claims, **characterized in that** the cationic associative polymer is chosen from quaternized cellulose derivatives, cationic polyurethanes, cationic polyvinyllactams and cationic acrylic terpolymers.

15. Composition according to any one of the preceding claims, **characterized in that** the content of cationic associative polymer represents from 0.01% to 5% by weight relative to the weight of the dye composition.

16. Composition according to any one of the preceding claims, **characterized in that** the oxidation dye precursor is chosen from oxidation bases and couplers, or mixtures thereof.

17. Composition according to Claim 12, **characterized in that** the content of oxidation base represents from 0.0005% to 12% by weight relative to the weight of the dye composition.

18. Composition according to Claim 12, **characterized in that** the coupler content represents from 0.0001% to 15% by weight relative to the weight of the dye composition.

19. Composition according to any one of the preceding claims, **characterized in that** the content of direct dye represents from 0.0005% to 15% by weight relative to the weight of the dye composition.

20. Composition according to any one of the preceding claims, **characterized in that** the composition comprises at least one basifying agent.

21. Composition according to the preceding claim, **characterized in that** the basifying agent is chosen from aqueous ammonia, alkanolamines and combinations of C₂-C₁₀ alkanolamines with alkali metal or alkaline-earth metal silicates.

22. Composition according to any one of the preceding claims, **characterized in that** it comprises at least one cationic or amphoteric substantive polymer.

23. Composition according to the preceding claim, **characterized in that** the content of cationic or amphoteric substantive polymer represents from 0.01% to 10% by weight relative to the weight of the dye composition.

24. Composition according to any one of the preceding claims, **characterized in that** it comprises at least one oxidizing agent.

25. Process for dyeing keratin fibres, in which a dye composition according to any one of Claims 1 to 24 is applied to the said wet or dry fibres.

26. Process for dyeing keratin fibres, in which a dye composition according to any one of Claims 1 to 23 is applied to the said wet or dry fibres, in the presence of an oxidizing composition, which is applied simultaneously with or successively to the dye composition without intermediate rinsing, the mixture is left on the fibres, and the fibres are rinsed.

27. Process for dyeing keratin fibres, in which a dye composition according to any one of Claims 1 to 23 is applied to the said wet or dry fibres, in the presence of an oxidizing composition, which is mixed with the dye composition before application, the mixture is left on the fibres, and the fibres are rinsed.

28. Multi-compartment device that may be used for dyeing keratin fibres, comprising a first compartment containing a composition according to any one of Claims 1 to 23, and comprising a second compartment containing an oxidizing composition

## Patentansprüche

1. Färbezusammensetzung, die in einem zum Färben von Keratinfasern geeigneten Medium enthält:
• mindestens einen Vorläufer eines Oxidationsfarbstoffs und/oder mindestens einen Direktfarbstoff;
• mindestens ein nichtionisches oder anionisches Tensid oder Gemische davon;
• mindestens Hydroxyethylcellulose;
• mindestens ein kationisches Assoziativpolymer;
• mindestens einen Fettstoff;
• wobei das Gewichtsverhältnis der Gesamtmenge der nichtionischen und anionischen Tenside zur Gesamtmenge an Fettstoff größer als oder gleich 1,75 ist, und
• wobei der Wassergehalt in der Färbezusammensetzung mindestens 40 Gew.-% beträgt, bezogen auf das Gewicht der Färbezusammensetzung.

2. Zusammensetzung nach dem vorhergehenden Anspruch, **dadurch gekennzeichnet, dass** der Wassergehalt mindestens 45 Gew.-% beträgt, bezogen auf das Gewicht der Färbezusammensetzung.

3. Zusammensetzung nach einem der vorhergehenden Ansprüche, **dadurch gekennzeichnet, dass** der Wassergehalt mindestens 50 Gew.-% beträgt, bezogen auf das Gewicht der Färbezusammensetzung.

4. Zusammensetzung nach einem der vorhergehenden Ansprüche, **dadurch gekennzeichnet, dass** das nichtionische Tensid ausgewählt ist unter:
• oxyalkylenierten oder glycerylierten Fettalkoholen;
• oxyalkylenierten Alkylphenolen mit einer C₈-C₁₈-Alkylkette;
• oxyalkylenierten oder glycerylierten Fettamiden;
• oxyalkylenierten Pflanzenölen;
• gegebenenfalls oxyalkylenierten Sorbitan-Fettsäureestern;
• gegebenenfalls oxyalkylenierten Saccharose-Fettsäureestern;
• Fettsäureestern von Polyethylenglycol;
• C₆-C₃₀-Alkylpolyglycosiden;
• N-(C₆-C₃₀)-Alkylglucamin-Derivaten;
• Aminoxiden wie Oxiden von C₁₀-C₁₄-Alkylaminen oder Oxiden von N-Acylaminopropylmorpliolinen;
• Copolymeren von Ethylenoxid und Propylenoxid;
• Gemischen der Verbindungen.

5. Zusammensetzung nach einem der vorhergehenden Ansprüche, **dadurch gekennzeichnet, dass** das nichtionische Tensid unter oxyalkylenierten oder glycerylierten Fettalkoholen ausgewählt ist.

6. Zusammensetzung nach einem der vorhergehenden Ansprüche, **dadurch gekennzeichnet, dass** das anionische Tensid ausgewählt ist unter:
• C₆-C₃₀₋Alkylsulfaten, C₆-C₃₀-Alkylethersulfaten, C₆-C₃₀-Alkylamidoethersulfaten, Alkylarylpolyethersulfaten, Monoglyceridsulfaten;
• C₆-C₃₀-Alkylsulfonaten, C₆-C₃₀-Alkylamidosulfonaten, C₆-C₃₀-Alkyl-arylsulfonaten, α-Olefinsulfonaten, Paraffinsulfonaten;
• C₆-C₃₀-Alkylphosphaten;
• C₆-C₃₀-Alkylsulfosuccinaten, C₆-C₃₀-Alkylethersulfosuccinaten, C₆-C₃₀-Alkylamidosulfosuccinaten;
• C₆-C₃₀-Alkylsulfoacetaten;
• C₆-C₂₄-Acylsarcosinaten;
• C₆-C₂₄-Acylglutamaten;
• Ethern von C₆-C₃₀-Alkylpolyglycosid-carbonsäuren; C₆-C₃₀-Alkylpolyglycosid-sulfosuccinaten;
• C₆-C₃₀-Alkylsulfosuccinamaten;
• C₆-C₂₄-Acylisethionaten;
• N-(C₆-C₂₄)-acyltauraten;
• C₆-C₃₀-Fettsäuren; Salzen von Säuren von Coprahöl oder hydriertem Coprahöl;
• C₈-C₂₀-Acyllactylaten;
• Salzen von C₆-C₃₀-Alkyl-D-galactosiduronsäuren;
• Salzen von polyoxyalkylenierten C₆-C₃₀-Alkylethercarbonsäuren, polyoxyalkylenierten C₆-C₃₀-Alkyl-arylethercarbonsäuren, polyoxyalkylenierten C₆-C₃₀-Alkyl-amidoethercarbonsäuren
• sowie Gemischen dieser Verbindungen.

7. Zusammensetzung nach einem der vorhergehenden Ansprüche, **dadurch gekennzeichnet, dass** der Gesamtgehalt an nichtionischem und/oder anionischem Tensid 0,01 bis 50 Gew.-% beträgt, bezogen auf das Gewicht der Färbezusammensetzung.

8. Zusammensetzung nach einem der vorhergehenden Ansprüche, **dadurch gekennzeichnet, dass** der Fettstoff ausgewählt ist unter nicht-oxyalkylenierten und nicht-glycerylierten Fettalkoholen, Amiden von nicht-oxyalkylenierten und nicht-glycerylierten Fettsäuren, Mono- und Polyestern von Carbonsäuren, Mineralölen, Pflanzenölen oder Gemischen dieser Stoffe.

9. Zusammensetzung nach einem der vorhergehenden Ansprüche, **dadurch gekennzeichnet, dass** der Gehalt an Fettstoffen höchstens 10 % beträgt, bezogen auf das Gewicht der Färbezusammensetzung.

10. Zusammensetzung nach einem der vorhergehenden Ansprüche, **dadurch gekennzeichnet, dass** das Gewichtsverhältnis der Gesamtmenge an nichtionischen und anionischen Tensiden zur Menge an Fettstoffen größer als 2 ist.

11. Zusammensetzung nach einem der vorhergehenden Ansprüche, **dadurch gekennzeichnet, dass** das Gewichtsverhältnis der Gesamtmenge an nichtionischen und anionischen Tensiden zur Menge an Fettstoffen kleiner als 20 ist.

12. Zusammensetzung nach einem der vorhergehenden Ansprüche, **dadurch gekennzeichnet, dass** die Hydroxyethylcellulose ein mittleres Molekulargewicht von mindestens 700000 aufweist.

13. Zusammensetzung nach einem der vorhergehenden Ansprüche, **dadurch gekennzeichnet, dass** der Gehalt an Hydroxyethylcellulose 0,01 bis 5 Gew.-% beträgt, bezogen auf das Gewicht der Färbezusammensetzung.

14. Zusammensetzung nach einem der vorhergehenden Ansprüche, **dadurch gekennzeichnet, dass** das kationische assoziative Polymer ausgewählt ist unter Derivaten quaternisierter Cellulose, kationischen Polyurethanen, kationischen Polyvinyllactamen und kationischen Acrylterpolymeren.

15. Zusammensetzung nach einem der vorhergehenden Ansprüche, **dadurch gekennzeichnet, dass** der Gehalt an kationischem assoziativem Polymer 0,01 bis 5 Gew.-% beträgt, bezogen auf das Gewicht der Färbezusammensetzung.

16. Zusammensetzung nach einem der vorhergehenden Ansprüche, **dadurch gekennzeichnet, dass** der Vorläufer des Oxidationsfarbstoffs unter Oxidationsbasen, Kupplern oder Gemischen davon ausgewählt ist.

17. Zusammensetzung nach Anspruch 12, **dadurch gekennzeichnet, dass** der Gehalt an Oxidationsbase 0,0005 bis 12 Gew.-% beträgt, bezogen auf das Gewicht der Färbezusammensetzung.

18. Zusammensetzung nach Anspruch 12, **dadurch gekennzeichnet, dass** der Gehalt an Kuppler 0,0001 bis 15 Gew.-% beträgt, bezogen auf das Gewicht der Färbezusammensetzung.

19. Zusammensetzung nach einem der vorhergehenden Ansprüche, **dadurch gekennzeichnet, dass** der Gehalt an Direktfarbstoff 0,0005 bis 15 Gew.-% beträgt, bezogen auf das Gewicht der Färbezusammensetzung.

20. Zusammensetzung nach einem der vorhergehenden Ansprüche, **dadurch gekennzeichnet, dass** die Zusammensetzung mindestens ein Mittel zum Alkalischmachen enthält.

21. Zusammensetzung nach dem vorhergehenden Anspruch, **dadurch gekennzeichnet, dass** das Mittel zum Alkalischmachen ausgewählt ist unter Ammoniak, Alkanolaminen und Kombinationen von C₂-C₁₀-Alkanolaminen mit Alkalimetallsilicaten oder Erdalkalimetallsilicaten.

22. Zusammensetzung nach einem der vorhergehenden Ansprüche, **dadurch gekennzeichnet, dass** sie mindestens ein kationisches oder amphoteres substantives Polymer enthält.

23. Zusammensetzung nach dem vorhergehenden Anspruch, **dadurch gekennzeichnet, dass** der Gehalt an kationischem oder amphoterem substantivem Polymer 0,01 bis 10 Gew.-% beträgt, bezogen auf das Gewicht der Färbezusammensetzung.

24. Zusammensetzung nach einem der vorhergehenden Ansprüche, **dadurch gekennzeichnet, dass** sie mindestens ein Oxidationsmittel enthält.

25. Verfahren zum Färben von Keratinfasern, bei dem auf die trockenen oder feuchten Fasern eine Färbezusammensetzung nach einem der Ansprüche 1 bis 24 aufgebracht wird.

26. Verfahren zum Färben von Keratinfasern, bei dem auf die trockenen oder feuchten Keratinfasern eine Färbezusammensetzung nach einem der Ansprüche 1 bis 23 in Gegenwart einer oxidierenden Zusammensetzung aufgebracht wird, die gleichzeitig mit der Färbezusammensetzung oder danach ohne Zwischenspülen aufgebracht wird, einwirken gelassen wird und dann die Fasern gespült werden.

27. Verfahren zum Färben von Keratinfasern, bei dem auf die trockenen oder feuchten Keratinfasern eine Färbezusammensetzung nach einem der Ansprüche 1 bis 23 in Gegenwart einer oxidierenden Zusammensetzung aufgebracht wird, die vor dem Aufbringen mit der Färbezusammensetzung gemischt wurde, einwirken gelassen wird und dann die Fasern gespült werden.

28. Vorrichtung mit mehreren Compartments zur Verwendung zum Färben von Keratinfasern, die ein erstes Compartment, das eine Zusammensetzung nach einem der Ansprüche 1 bis 23 enthält, und ein zweites Compartment aufweist, das eine oxidierende Zusammensetzung enthält.
